# EUROPÄISCHE PATENTSCHRIFT

(11) **EP 0 596 361 B1**
(45) Veröffentlichungstag und Bekanntmachung des Hinweises auf die Patenterteilung: **27.08.1997**
(21) Anmeldenummer: 93117242.3
(22) Anmeldetag: 25.10.1993
(51) Int. Cl.: C07C 43/12, C07C 41/01, C07D 235/10, C07D 491/04

(54) **Chlor-(2-halogen-1-fluor-methyl-ethoxy)-methane und deren Herstellung**
Chloro-(2-halo-1-fluoromethyl-ethoxy) methanes and their preparation
Méthanes chloro-(halo-2-fluorométhyl-1-éthoxy) et leur préparation

(30) Priorität: 06.11.1992 DE 4237556
(43) Veröffentlichungstag der Anmeldung: 11.05.1994
(73) Patentinhaber: BAYER AG, 51368 Leverkusen (DE)
(72) Erfinder: Böhm, Stefan, Dr., D-51065 Köln (DE); Marhold, Albrecht, Dr., D-51373 Leverkusen (DE)

(56) Entgegenhaltungen:
- EP-A- 0 320 981
- DE-A- 3 636 386
- FR-A- 1 469 504
- US-A- 3 749 789

## Beschreibung

Die vorliegende Erfindung betrifft neue Chlor-(2-halogen-1-fluormethyl-ethoxy)-methane und deren Herstellung aus den entsprechenden halogenierten Isopropanolen. Die neuen Verbindungen eignen sich zur Herstellung von biologisch wirksamen substituierten Benzimidazolen.

Es ist bekannt, daß man Isopropanol chlormethylieren und so Chlor-1-methyl-ethoxymethane herstellen kann (siehe C.A. 73 (19): 98332 q). Werden im Isopropanol die Methylgruppen durch CH₂Hal-Gruppen ersetzt, so ist damit zu rechnen, daß die elektronegativen Halogenatome die Reaktivität der OH-Gruppe im Vergleich zu halogenfreiem Isopropanol herabsetzen und deshalb Chlor-(2-halogen-1-fluormethyl-ethoxy)-methane nicht oder nur sehr schlecht zugänglich sein sollten.

Es wurden nun Chlor-(2-halogen-1-fluormethyl-ethoxy)-methane der Formel (I) gefunden 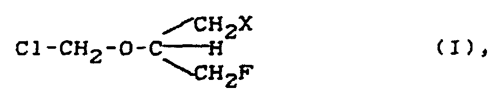 in der
- X: für Fluor oder Chlor steht.

Im einzelnen handelt es sich dabei um Chlor-(2-fluor-1-fluormethyl-ethoxy)-methan(Formel (I), X = Fluor) und Chlor-(2-chlor-1-fluormethyl-ethoxy)-methan (Formel (I), X = Chlor).

Das erfindungsgemäße Verfahren zur Herstellung von Verbindungen der Formel (I) ist dadurch gekennzeichnet, daß man halogenierte Isopropanole der Formel (II) 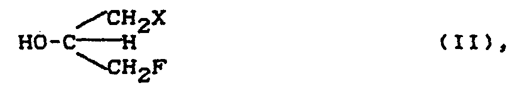 in der
- X: für Fluor oder Chlor steht,
bei -20 bis +20°C mit Formaldehyd und Chlorwasserstoff umsetzt.

Halogenierte Isopropanole der Formel (II) sind bekannte Verbindungen (siehe J. Chem. Soc. 1958, 2251).

Bevorzugte Reaktionstemperaturen für das erfindungsgemäße Verfahren sind solche im Bereich -10 bis ±0°C.

Das erfindungsgemäße Verfahren kann in Gegenwart von Lösungsmitteln durchgeführt werden, beispielsweise in Gegenwart von Kohlenwasserstoffen oder halogenierten Kohlenwasserstoffen, die bei Reaktionstemperatur flüssig sind. Vorzugsweise wird jedoch ohne Zusatz von Lösungsmitteln gearbeitet.

Der Formaldehyd kann in verschiedener Form, beispielsweise als Paraformaldehyd eingesetzt werden. Bezogen auf 1 Mol einer Verbindung der Formel (II) kann man beispielsweise 0,8 bis 1,5 Mol Formaldehyd einsetzen. Bevorzugt werden 1,0 bis 1,2 Mol Formaldehyd pro Mol einer Verbindung der Formel (II) eingesetzt.

Chlorwasserstoff wird vorzugsweise in gasförmiger Form eingesetzt. Man kann beispielsweise so verfahren, daß man in ein Gemisch aus einer Verbindung der Formel (II) und Formaldehyd oder Paraformaldehyd bei Reaktionstemperatur so lange gasförmigen Chlorwasserstoff einleitet, bis das Reaktionsgemisch zwei klare Phasen bildet.

Die Aufarbeitung des Reaktionsgemisches kann z.B. so erfolgen, daß man die organische Phase abtrennt, trocknet und, vorzugsweise im Vakuum, fraktioniert destilliert.

Man kann auf diese Weise Verbindungen der Formel (I) in Ausbeuten von deutlich über 50 % erhalten. Angesichts der eingangs geschilderten Verhältnisse (durch die Einführung von Halogenmethylgruppen am Isopropanol zu erwartende stark herabgesetzte Reaktionsfähigkeit der OH-Gruppe) ist dies ausgesprochen überraschend.

Zur Herstellung von biologisch wirksamen substituierten Benzimidazolen kann man beispielsweise so verfahren, daß man Verbindungen der Formel (I) mit einem Benzimidazolderivat der Formel (III) umsetzt 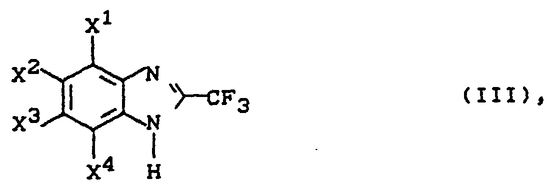 in der
- X¹, X², X³ und X⁴: unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder methylsulfonyl stehen, wobei jedoch mindestens einer der Substituenten X¹, X², X³ oder X⁴ für Halogenalkyl mit Ausnahme des Chlormethylrestes, Halogenalkoxy, Halogenalkylthio, Halogenalkylsulfinyl, Halogenalkylsulfonyl, Alkylsulfonyl, für gegebenenfalls substituiertes ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Arylthio, Arylsulfinyl, Arylsulfonyl, Arylsulfonyloxy, Arylcarbonyl, Aryloxycarbonyl, Arylazo oder Arylthiomethylsulfonyl stehen,
und so substituierte Benzimidazole der Formel (IV) erhalten 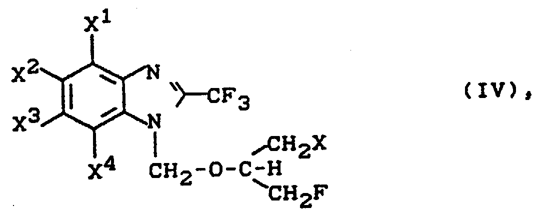 in der
- X: für Fluor oder Chlor stehen und
- X¹ bis X⁴: die bei Formel (III) angegebene Bedeutung haben.

In den Formeln (III) und (IV) stehen X¹ bis X⁴ bevorzugt unabhängig voneinander jeweils für Wasserstoff, Fluor, Chlor, Brom, Iod, Cyano, Nitro, für jeweils geradkettiges oder verzweigtes Alkyl, Alkoxy, Alkylthio, Alkylsulfinyl oder Alkylsulfonyl mit jeweils 1 bis 8 Kohlenstoffatomen, für Cycloalkyl mit 3 bis 8 Kohlenstoffatomen, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 5 Kohlenstoffatomen stehen, außerdem für Hydroxycarbonyl, für jeweils geradkettiges oder verzweigtes Alkylcarbonyl oder Alkoxycarbonyl mit jeweils 1 bis 6 Kohlenstoffatomen im Alkylteil, für Cycloalkyloxycarbonyl mit 3 bis 8 Kohlenstoffatomen im Cycloalkylteil oder für jeweils gegebenenfalls einfach oder mehrfach gleich oder verschieden substituiertes Amino oder Aminocarbonyl stehen, wobei als Aminosubstituenten jeweils in Frage kommen:
jeweils geradkettiges oder verzweigtes Alkyl mit 1 bis 6 Kohlenstoffatomen, Halogenalkyl mit 1 bis 6 Kohlenstoffatomen und 1 bis 13 Halogenatomen, Alkoxyalkyl oder Alkylcarbonyl mit jeweils 1 bis 6 Kohlenstoffatomen in den einzelnen Alkylteilen oder im Arylteil jeweils gegebenenfalls einfach oder mehrfach, gleich oder verschieden substituiertes Arylcarbonyl, Arylsulfonyl, Arylaminocarbonyl oder Arylmethylsulfonyl mit jeweils 6 bis 10 Kohlenstoffatomen im Arylteil, wobei gegebenenfalls vorhandene Arylsubstituenten ein- oder mehrfache, gleiche oder verschiedene Halogene und/oder geradkettiges oder verzweigtes Alkyl mit 1 bis 6 C-Atomen und/oder geradkettiges oder verzweigtes Halogenalkyl mit 1 bis 6 C-Atomen und 1 bis 13 gleichen oder verschiedenen Halogenen sein können und Aryl vorzugsweise für Phenyl steht;
außerdem für jeweils gegebenenfalls im Arylteil einfach oder mehrfach, gleich oder verschieden substituiertes Aryl, Aryloxy, Arylthio, Arylsulfinyl, Arylsulfonyl, Arylsulfonyloxy, Arylcarbonyl, Aryloxycarbonyl, Arylthiomethylsulfonyl oder Arylazo mit jeweils 6 bis 10 Kohlenstoffatomen im Arylteil stehen, wobei als Arylsubstituenten die oben genannten in Frage kommen und Aryl vorzugsweise für Phenyl steht und
wobei mindestens einer der Substituenten X¹, X², X³ oder X⁴ für jeweils geradkettiges oder verzweigtes Halogenalkyl (mit Ausnahme des Chlormethylrestes), Halogenalkoxy, Halogenalkylthio, Halogen-alkylsulfinyl, genalkylsulfonyl mit jeweils 1 bis 6 Kohlenstoffatomen und 1 bis 13 gleichen oder verschiedenen Halogenatomen, für geradkettiges oder verzweigtes Alkylsulfonyl mit 1 bis 6 Kohlenstoffatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 5 Kohlenstoffatomen steht, außerdem für Hydroxycarbonyl, für jeweils geradkettiges oder verzweigtes Alkylcarbonyl oder Alkoxycarbonyl mit jeweils 1 bis 6 Kohlenstoffatomen im Alkylteil, für Cycloalkyloxycarbonyl mit 3 bis 8 Kohlenstoffatomen im Cycloalkylteil oder für jeweils gegebenenfalls einfach oder mehrfach, gleich oder verschieden substituiertes Amino oder Aminocarbonyl steht, wobei als Aminosubstituenten jeweils in Frage kommen:
jeweils geradkettiges oder verzweigtes Alkyl mit 1 bis 6 Kohlenstoffatomen, Halogenalkyl mit 1 bis 6 Kohlenstoffatomen und 1 bis 13 Halogenatomen, Alkoxyalkyl oder Alkylcarbonyl mit jeweils 1 bis 6 Kohlenstoffatomen in den einzelnen Alkylteilen oder im Arylteil jeweils gegebenenfalls einfach der mehrfach, gleich oder verschieden substituiertes Arylcarbonyl, Arylsulfonyl, Arylaminocarbonyl oder Arylmethylsulfonyl mit jeweils 6 bis 10 Kohlenstoffatomen im Arylteil, wobei als Arylsubstituenten die oben genannten in Frage kommen und Aryl vorzugsweise für Phenyl steht;
außerdem für jeweils gegebenenfalls im Arylteil einfach oder mehrfach, gleich oder verschieden substituiertes Aryl, Arylthio, Arylsulfinyl, Arylsulfonyl, Arylsulfonyloxy, Arylcarbonyl, Aryloxycarbonyl, Arylthiomethylsulfonyl oder Arylazo mit jeweils 6 bis 10 Kohlenstoffatomen im Arylteil steht, wobei als Arylsubstituenten die oben genannten in Frage kommen und Aryl vorzugsweise für Phenyl steht.

Besonders bevorzugt steht in den Formeln (III) und (IV)
- X¹: für Wasserstoff,
- X²: für CF₃ oder OCF₃,
- X³: für Chlor oder OCF₃ oder
- X² und X³: gemeinsam für -O-CF₂-O-, -O-CF₂-CF₂-O- oder -O-CF₂-CHF-O- und
- X⁴: für Wasserstoff.

Benzimidazole der Formel (III) sind teilweise bekannt (siehe J. Am. Chem. Soc. 75, 1292 (1953) und US-PS 3 576 818). Dioxolgruppierungen des Typs 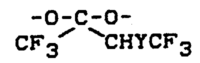 mit Y = H oder Hal enthaltende Benzimidazole der Formel (III) können beispielsweise erhalten werden durch Umsetzung von entsprechenden Dihydroxybenzolen mit 1,1,1,4,4,4-Hexafluor-2-butenen der Formel (V) 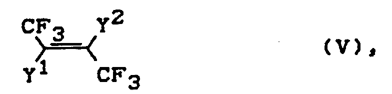 in der
- Y¹: für H oder Halogen und
- Y²: für Halogen steht,
in Gegenwart einer Base und eines Verdünnungsmittels bei -20 bis +200°C.

Substituierte Benzimidazole der Formel (IV) sind beispielsweise geeignet zur Bekämpfung von tierischen Schädlingen wie Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Die Herstellung von substituierten Benzimidazolen der Formel (IV), solche Benzimidazole als Stoffe, sie enthaltende Schädlingsbekämpfungsmittel, deren Herstellung, Dioxolgruppen des Typs 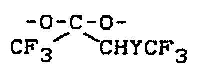 mit Y = H oder Hal enthaltende Benzimidazole der Formel (III) und deren Herstellung sind Gegenstand anderer Schutzrechtsanmeldungen des gleichen Anmelders.

### Beispiel

192 g 1,3-Difluor-2-propanol wurden mit 66 g Paraformaldehyd (fein gepulvert) versetzt. Dann wurde bei -10°C ein kräftiger Chlorwasserstoff-Gasstrom unter Rühren eingeleitet, bis eine klare 2-phasige Mischung entstanden war. Anschließend wurde die organische Phase abgetrennt, mit Calciumchlorid getrocknet und im Vakuum fraktioniert destilliert. Mit einem Siedepunkt von 50 bis 54°C bei 20 mbar wurden 183 g (60 % der Theorie) Chlor-(2-fluor-1-fluormethyl-ethoxy)-methan erhalten. Die charakteristischen Absorptionen in den NMR-Spektren waren wie folgt:
¹H-NMR: 5,6 ppm und 4,55 ppm.
¹⁹F-NMR: -233 ppm.

## Patentansprüche

1. Chlor-(2-halogen-1-fluormethyl-ethoxy)-methane der Formel (I) 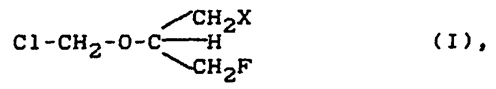 in der
X für Fluor oder Chlor steht.

2. Verbindungen nach Anspruch 1, dadurch gekennzeichnet, daß es sich um Chlor-(2-fluor-1-fluormethyl-ethoxy)-methan oder Chlor-(2-chlor-1-fluormethyl-ethoxy)-methan handelt.

3. Verfahren zur Herstellung der Verbindungen des Anspruchs 1, dadurch gekennzeichnet, daß man halogenierte Isopropanole der Formel (II) 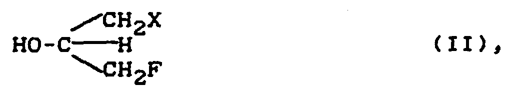 in der
X für Fluor oder Chlor steht,
bei -20 bis +20°C mit Formaldehyd und Chlorwasserstoff umsetzt.

4. Verfahren nach Anspruch 3, dadurch gekennzeichnet, daß man bei -10 bis ±0°C arbeitet.

5. Verfahren nach den Ansprüchen 3 und 4, dadurch gekennzeichnet, daß man 0,8 bis 1,5 Mol Formaldehyd pro Mol der Verbindung der Formel (II) einsetzt.

6. Verfahren nach Ansprüchen 1 bis 5, dadurch gekennzeichnet, daß man Formaldehyd in Form von formaldehyd einsetzt.

7. Verfahren nach Ansprüchen 3 bis 6, dadurch gekennzeichnet, daß man den Chlorwasserstoff gasförmig einsetzt.

## Claims

1. Chloro- (2-halogeno-1-fluoromethyl-ethoxy) - methanes of formula (I) 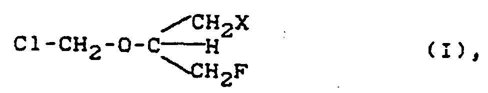 in which
X is fluorine or chlorine.

2. Compounds according to Claim 1, characterised in that they are chloro-(2-fluoro-1-fluoromethyl-ethoxy)-methane or chloro-(2-chloro-1-fluoromethyl-ethoxy)-methane.

3. Process for the preparation of the compounds of Claim 1, characterised in that halogenated isopropanols of formula (II) 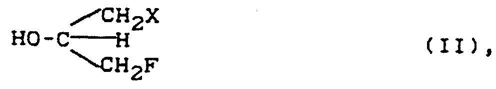 in which
X is fluorine or chlorine,
are reacted with formaldehyde and hydrogen chloride at -20 to +20°C.

4. Process according to Claim 3, characterised in that it is carried out at -10 to ±0°C.

5. Process according to Claims 3 and 4, characterised in that 0.8 to 1.5 mol of formaldehyde are used per mole of compound of formula (II).

6. Process according to Claims 1 to 5, characterised in that the formaldehyde is used in the form of paraformaldehyde.

7. Process according to Claims 3 to 6, characterised in that the hydrogen chloride is used in gaseous form.

## Revendications

1. Chloro-(2-halogéno-1-fluorométhyléthoxy)-méthanes de formule (I): 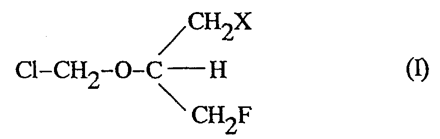 dans laquelle
X représente le fluor ou le chlore.

2. Composés selon la revendication 1, caractérisés en ce qu'il s'agit du chloro-(2-fluoro-1-fluorométhyléthoxy)-méthane ou du chlore-(2-chlore-1-fluorométhyléthoxy)-méthane.

3. Procédé de préparation des composés selon la revendication 1, caractérisé en ce que l'on fait réagir des isopropanols halogénés de formule (II) : 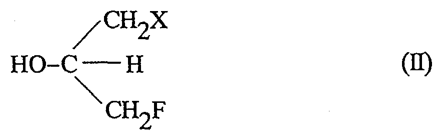 dans laquelle
X représente le fluor ou le chlore,
entre -20 et +20°C avec du formaldéhyde et du chlorure d'hydrogène.

4. Procédé selon la revendication 3, caractérisé en ce que l'on opère entre -10 et ±0°C.

5. Procédé selon les revendications 3 et 4, caractérisé en ce que l'on utilise 0,8 à 1,5 mol de formaldéhyde par mole du composé de formule (II).

6. Procédé selon les revendications 1 à 5, caractérisé en ce que l'on utilise du formaldéhyde sous forme de paraformaldéhyde.

7. Procédé selon les revendications 3 à 6, caractérisé en ce que l'on utilise le chlorure d'hydrogène sous forme gazeuse.
